# EUROPÄISCHE PATENTSCHRIFT

(11) **EP 3 612 835 B1**
(45) Veröffentlichungstag und Bekanntmachung des Hinweises auf die Patenterteilung: **17.08.2022**
(21) Anmeldenummer: 18723663.3
(22) Anmeldetag: 20.04.2018
(51) Int. Cl.: G01N 33/02, B07C 5/342, G01N 21/85, G01N 33/03

(54) **VERFAHREN ZUR DETEKTION DER RANZIGKEIT VON ÖLFRÜCHTEN, SAMEN UND NÜSSEN**
METHOD FOR DETECTING THE RANCIDITY OF OILSEEDS, SEEDS AND NUTS
PROCÉDÉ PERMETTANT DE DÉTECTER LA RANCIDITÉ DE FRUITS OLÉAGINEUX, DE GRAINES ET DE NOIX

(30) Priorität: 21.04.2017 AT 503222017
(43) Veröffentlichungstag der Anmeldung: 26.02.2020
(73) Patentinhaber: Insort GmbH, 8324 Kirchberg an der Raab (AT); Technische Universität Graz, 8010 Graz (AT)
(72) Erfinder: PALTAUF, Günther Wolfgang, 8010 Graz (AT); LEITNER, Erich, 8010 Graz (AT)
(74) Vertreter: Margotti, Herwig Franz
(86) Internationale Anmeldenummer: PCT/AT2018/060075
(87) Internationale Veröffentlichungsnummer: WO 2018/191768

(56) Entgegenhaltungen:
- EP-B1- 1 332 354
- US-A1- 2013 278 919
- ARANTZAZU VALDÉS ET AL: "Monitoring the oxidative stability and volatiles in blanched, roasted and fried almonds under normal and accelerated storage conditions by DSC, thermogravimetric analysis and ATR-FTIR : Effect of cooking on the oxidative stability of almonds", EUROPEAN JOURNAL OF LIPID SCIENCE AND TECHNOLOGY., Bd. 117, Nr. 8, 24. Februar 2015 (2015-02-24), Seiten 1199-1213, XP055494712, DE ISSN: 1438-7697, DOI: 10.1002/ejlt.201400384
- BELTRN A ET AL: "Monitoring the oxidation of almond oils by HS-SPMEGCMS and ATR-FTIR: Application of volatile compounds determination to cultivar authenticity", FOOD CHEMISTRY, ELSEVIER LTD, NL, Bd. 126, Nr. 2, 10. November 2010 (2010-11-10), Seiten 603-609, XP028211008, ISSN: 0308-8146, DOI: 10.1016/J.FOODCHEM.2010.11.058 [gefunden am 2010-11-16] in der Anmeldung erwähnt
- BORRÀS EVA ET AL: "Olive oil sensory defects classification with data fusion of instrumental techniques and multivariate analysis (PLS-DA)", FOOD CHEMISTRY, ELSEVIER LTD, NL, Bd. 203, 4. Februar 2016 (2016-02-04), Seiten 314-322, XP029452314, ISSN: 0308-8146, DOI: 10.1016/J.FOODCHEM.2016.02.038

## Beschreibung

Die Erfindung betrifft ein Verfahren zum Erstellen einer Ranzigkeitsindextabelle und Zuordnen eines Ranzigkeitsindexwerts zu einem Absorptions- oder Reflektionsspektrums von Ölfrüchten, Nüssen und Samen. Des Weiteren betrifft sie ein Verfahren zur Detektion der Ranzigkeit einer Ölfrucht, Nuss oder eines Samens.

Die Erfindung betrifft weiters eine Vorrichtung zur Detektion ranziger Ölfrüchte, Nüsse oder Samen.

Die Detektion und anschließende Sortierung von Schüttgütern mithilfe von Fotosensoren ist eine gängige Methode. Eine Ausführungsform eines solchen Verfahrens und einer solchen Vorrichtung zur Sortierung von Samen wird beispielsweise in der Veröffentlichung US 2013/0278919 A1 beschrieben. Bei diesem bekannten Verfahren werden Samen einzeln spektroskopisch untersucht, indem sie mit einer Lichtquelle bestrahlt werden. Anschließend wird ein Absorptions- oder Reflektionsspektrum von einem Fotosensor aufgenommen. Eine Rechnereinheit analysiert daraufhin das Absorptions- oder Reflektionsspektrums jedes Samens in einem Bereich von Interesse und errechnet anhand einer Kalibrierkurve den Gehalt eines bestimmten Inhaltsstoffes des Samens.

Das Dokument ARANTZAZU VALDES ET AL: "Monitoring the oxidative stability and volatiles in blanched, roasted and fried almonds under normal and accelerated storage conditions by DSC, thermogravimetric analysis and ATR-FTIR : Effect of cooking on the oxidative stability of almonds", EUROPEAN JOURNAL OF LIPID SCIENCE AND TECHNOLOGY., Bd. 117, Nr. 8, 24. Februar 2015, Seiten 1199-1213, XP055494712, ISSN: 1438-7697, D01: 10.1002/ejlt.201400384 offenbart ein Verfahren zum Erstellen einer Ranzigkeitsindextabelle gemäß dem Oberbegriff des Anspruchs 1 beziehungsweise ein Verfahren gemäß dem Oberbegriff von Anspruch 11.

Das Dokument BORRÀS EVA ET AL: "Olive oil sensory defects classification with data fusion of instrumental techniques and multivariate analysis (PLS-DA)", FOOD CHEMISTRY, ELSEVIER LTD, Bd. 203, 4. Februar 2016 (2016-02-04), Seiten 314-322, XP029452314, ISSN: 0308-8146, D01: 10.1016/J.FOODCHEM.2016.02.038 offenbart ein Verfahren zur Klassifizierung sensorischer Mängel bei Olivenöl mittels Datenfusion instrumenteller Techniken und multivarianter Analyse.

Der Nachweis verschiedener Inhaltsstoffe in einzelnen Elementen eines Schüttguts ist von Interesse, um beispielsweise eine Unterscheidung zwischen verdorbenen Elementen und nicht verdorbenen Elementen des Schüttguts treffen zu können. Gemäß dem Stand der Technik arbeiten derartige Verfahren in der Regel im Nahinfrarotbereich. Um einen Einsatz dieser Verfahren in Produktionsanlagen zu ermöglichen ist es notwendig, dass die eingesetzten Fotosensoren hohe Bildwiederholraten, in der Regel von 300 Hz oder mehr aufweisen. Somit wird ermöglicht einen hohen Durchsatz bei gleichzeitig zuverlässiger Analyse der Inhaltsstoffe jedes einzelnen untersuchten Elements zu gewährleisten. Herkömmlicherweise werden von den Fotosensoren erfasste Daten mit gängigen statistischen Klassifikationsmethoden wie Partial Least Squares, Principle Component Regression oder ähnlichem analysiert. Diese qualitative Analyse führt zu sehr guten Ergebnissen, wenn es klare Unterschiede zwischen verdorbenen Elementen und nicht verdorbenen Elementen im Absorptions- oder Reflektionsspektrum gibt.

Als nachteilig hat sich in derartigen Verfahren erwiesen, dass, wenn die Absorptions- oder Reflektionsspektra zu ähnlich werden, der Versuch einer Trennung zwischen verdorbenen Elementen und nicht verdorbenen Elementen in der Regel zu sehr vielen Fehlklassifikationen führt. Besonders nachteilig ist, dass im Falle einer nachfolgenden Sortierung dies zu sehr hohem Ausschuss von nicht verdorbenen Elementen führt. Gleichzeitig ergibt sich der Nachteil, dass nur eine sehr niedrige Detektionsrate von verdorbenen Elementen realisiert wird. Vor allem bei Naturprodukten wie Lebensmitteln ist dieser nachteilige Effekt besonders präsent, da die natürliche spektrale Streuung von nicht verdorbenen Elementen sehr groß im Vergleich zu kontrolliert hergestellten Elementen, wie beispielsweise Plastikflakes, ist.

Insbesondere bei Ölfrüchten, Nüsse und Samen, welche in Produktionsanlagen automatisiert verarbeitet werden, besteht ein großes Interesse ranzige, und somit verdorbene, Elemente von nicht ranzigen Elementen automatisiert zu unterschieden.

Die geschmackliche Qualität von Nüssen und anderen Samen oder Ölfrüchten wird oft durch Lipidoxidation beeinträchtigt, was zu unerwünschten ranzigen Aromen führt. Die Lipidoxidation von Nüssen und anderen Ölfrüchten mit einem hohen Fettgehalt und daraus resultierende Ranzigkeit kann im Zuge der Lagerung und Verarbeitung auftreten. Dies beeinträchtigt massiv die sensorischen Eigenschaften (Verminderung der Genussqualität) und führt zu einem wertgeminderten Produkt.

Die Mechanismen der Lipidoxidation sind in der Literatur gut bekannt und beschrieben. Es gibt zwei verschiedene Mechanismen, die Ranzigkeit verursachen. Hydrolytische Ranzigkeit wird durch die Reaktion von Wasser mit Lipiden in Gegenwart von enzymatischer Aktivität (Lipase) verursacht. Oxidative Ranzigkeit kann in autooxidative, photooxidative und enzymatische Oxidationsreaktionen unterschieden werden. Die Fettsäurezusammensetzung ist neben anderen Faktoren hinsichtlich der Stabilität des Produktes kritisch. Die Stabilität von ungesättigten Säuren nimmt durch die Erhöhung des Grades der Ungesättigtheit dramatisch ab. Die Oxidationsgeschwindigkeit der Fettsäuren beträgt etwa 1: 10: 100: 200 für Stearinsäure (18: 0), Ölsäure (18: 1, ω - 9), Linolsäure (18: 2, ω - 6) und α - Linolensäure (18: 3, ω - 3).

Für die Bestimmung der Ranzigkeit können verschiedene Methoden verwendet werden. Die meisten Methoden erfordern eine große Probenmenge, so dass eine homogene Mischung von gemahlenen Ölfrüchten, Nüssen bzw. Samen verwendet wird. Dies beinhaltet den Nachteil, dass durch Homogenisierung einer großen Anzahl von Ölfrüchten, Nüssen bzw. Samen wertvolle Informationen von einzelnen verloren gehen. Ein weiterer Nachteil der bekannten Methoden ist darin zu sehen, dass diese Methoden bloße Labormethoden sind, die sich nicht für die Anwendung in automatisierten Detektionsverfahren und Sortieranlagen eignen. Darüber hinaus sind diese Labormethoden enorm zeitaufwändig und, wie oben erwähnt, für homogenisierte Produkte, nicht aber für einzelne Ölfrüchte, Samen und Nüsse anwendbar. Aus dem Dokument A. Beltrán, M. Ramos, N. Grané, M.L. Martin, M.C. Garrigós, Monitoring the oxidation of almond oils by HS-SPME-GC-MS and ATR-FTIR: Application of volatile compounds determination to cultivar authenticity, Food Chemistry, Volume 126, lssue 2, 2011, Pages 603-609, ist ein Verfahren zur Bestimmung von flüchtigen Bestandteilen von Mandelölen zur Überprüfung der Anbauauthentizität von Mandeln bekannt. Genauer gesagt dient dieses Verfahren zur Unterscheidung zwischen spanischen und amerikanischen Mandelanbaukulturen und eventuellen Fälschungen. Dazu wird der oxidative Prozess dieser Öle mittels Festphasen Mikroextraktion/Gaschromatographie - Massenspektrometrie (HS-SPME/GC-MS) und Totalreflexions-Fouriertransformation-Infrarot-Spektroskopie (ATR-FTIR) überwacht. Für die Beschleunigung der Lipidoxidation wird eine Wärmebehandlung der Proben bei 100°C für 1, 3, 5, 7, 10, 15 und 20 Tage durchgeführt und die oxidative Stabilität der Proben nach diesen Wärmebehandlungen überprüft. In den Infrarot-Spaktralbändern beobachtete Veränderungen werden zur Überwachung des Fortschreitens der Oxidation von Mandelölen benutzt. Die Ranzigkeit von Mandeln wird nicht explizit erwähnt, sondern nur von "off-flavour development" gesprochen.

Diese Studie wurde nicht an einzelnen Mandelkernen, sondern an aus einer Vielzahl von Mandeln gewonnenem Mandelöl, d.h. einem homogenisierten Produkt, durchgeführt. Es ist weder erwähnt, noch angedeutet, wie mithilfe der Ergebnisse dieser Studie eine Detektion und gegebenenfalls Aussortierung von einzelnen Mandeln in einem Produktstrom bewerkstelligt werden könnte. Das Ziel der Studie war es vielmehr, ein Verfahren basierend auf HS-SPME gekoppelt mit GC-MS vorzuschlagen, mit dem die rasche Analyse und Charakterisierung von aus der Lipidoxidation resultierenden flüchtigen Komponenten in Mandelölen möglich sein soll. Als Ergänzung zur Messung mit HS-SPME/GC-MS wurden auch die ATR-FTIR Spektren von Mandelölen während des Oxidationsprozesses überwacht. Es ist erwähnt, dass bei der HS-SPME-GC-MS Analyse die optimale Zeit für die oxidative Wärmebehandlung der Proben sieben Tage beträgt, weil erst nach dieser Zeit signifikante Unterschiede im Aldehydgehalt erkennbar sind und diese Zeit eine "vernünftig kurze Zeitdauer" der Analysezeit darstellt. Was die zusätzliche Analyse durch ATR-FIR betrifft, so ist erläutert, dass die Proben nach Wärmebehandlungszeiten von 1 bis 20 Tagen unter oxidativen Bedingungen vermessen wurden, wobei nach Wärmebehandlungszeiten von einem, drei und fünf Tagen noch keine signifikanten Unterschiede in den erhaltenen Spektren gefunden wurden. Erst nach dem fünften Tag der Wärmebehandlung konnten spektrale Veränderungen beobachtet werden, die auf fortschreitende Oxidation der Proben hindeuteten. Die Messungen mit HS-SPME/GC-MS bzw. ATR-FTIR wurden unabhängig voneinander durchgeführt und die Messresultate aus beiden Messarten dienten lediglich zur Feststellung, ob das Ergebnis der jeweils anderen Messart plausibel ist. Die Ergebnisse der beiden Messarten werden jedoch nicht miteinander verknüpft. Insbesondere wird kein Ranzigkeitsindex oder ein anderer Index aus den Messergebnissen erstellt.

In dem Dokument Borräs E, Ferré J, Boqué R, Mestres M, Acenia L, Calvo A, Busto O. Prediction of olive oil sensory descriptors using instrumental data fusion and partial least squares (PLS) regression. Talanta. 2016 Aug 1;155:116-23. Epub 2016 Apr 20 ist die Verwendung von Massenspektrometrie (HS-MS), Fouriertransformation-Mittelinfrarot-Spektroskopie (FT-MIR) und UV-sichtbares Licht-Spektrophotometrie (UV-vis) beschrieben, um Olivenöl-Geschmacksparameter vorherzusagen. Aus den Messwerten von 343 Olivenölproben von vier aufeinanderfolgenden Ernten wurden multivariate Kalibrierungsmodelle unter Verwendung partieller kleinster Quadrate Regression erstellt. HS-MS und FT-MIR Ergebnisse wurden entweder einzeln ausgewertet oder - zur Verbesserung des Vorhersagemodells - mittels "Datafusion" verknüpft, das ist die Zusammenführung von zwei Ergebnismatritzen der beiden unterschiedlichen Messarten zu einer Matrix. Es stellte sich bei dieser Untersuchung aber heraus, dass selbst die "Datafusion" keine brauchbare Vorhersage für Ranzigkeit von Olivenöl liefern konnte, weil nur 10% (!) der Proben mit ranzigem Olivenöl korrekt detektiert werden konnten. Als Konsequenz aus diesem miserablen Ergebnis hielten die Autoren von D2 fest, dass es nicht möglich war, brauchbare Ranzigkeits-Modelle aus den Messdaten zu erstellen.

Der Erfindung liegt somit die Aufgabe zugrunde ein Verfahren bereitzustellen, welches die geschilderten Nachteile des Standes der Technik vermeidet und eine automatisierte Detektion der Ranzigkeit von einzelnen Ölfrüchten, Nüssen und Samen ermöglicht.

Erfindungsgemäß wird diese Aufgabenstellung durch ein Verfahren zum Erstellen einer Ranzigkeitsindextabelle und Zuordnen eines Ranzigkeitsindexwert zu einem Absorptions- oder Reflektionsspektrums von Ölfrüchten, Nüssen und Samen, umfassend die Schritte:
- Bestrahlen einer Probe einer Ölfrucht, einer Nuss oder eines Samens mit einer Lichtquelle,
- Projizieren des reflektierten und/oder transmittierten Lichts auf einen Fotosensor,
- Erfassen des Absorptions- oder Reflektionsspektrums der Probe in einem Wellenlängenbereich von 900 bis 2500 nm, vorzugsweise von 900 bis 1700 nm, noch bevorzugter von 1000 bis 1500nm, durch den Fotosensor,
- Extrahieren von Inhaltsstoffen der Probe durch Probenvorbereitungstechniken auf Basis einer Bestimmung flüchtiger Verbindungen aus einem Dampfraum über der Probe, vorzugsweise Festphasenmikroextraktion,
- Trennen von flüchtigen Komponenten der Probe mittels gaschromatographischer Techniken,
- Identifizieren von abgetrennten flüchtigen Komponenten der Probe durch massenspektroskopische Detektion von für die Lipidoxidation relevanten Bestandteilen der flüchtigen Komponenten,
- Bestimmen eines Ranzigkeitsindexwerts der Probe aus identifizierten flüchtigen Komponenten der Probe,
- Zuordnen zumindest von einzelnen charakteristischen Wellenlängen oder Wellenlängenbereichen des erfassten Absorptions- oder Reflektionsspektrums der Probe zu dem Ranzigkeitsindexwert,
- Wiederholen der vorangegangenen Schritte für eine repräsentative Anzahl an Proben und Bilden einer Ranzigkeitstabelle aus den ermittelten Ranzigkeitsindexwerten und zugeordneten Absorptions- oder Reflektionsspektren bzw. charakteristischen Wellenlängen oder Wellenlängenbereichen der erfassten Absorptions- oder Reflektionsspektren
   gelöst.

Im Unterschied zu analytischen, enorm zeitaufwändigen Methoden im Labor, wie den oben beschriebenen bekannten Verfahren, ist dieses erfindungsgemäße Verfahren geeignet, zur automatisierten Detektion und Sortierung von Ölfrüchten, Samen und Nüssen in Abhängigkeit ihres Ausmaßes an Ranzigkeit verwendet zu werden, wobei ein hoher Produktdurchsatz an einzelnen Früchten detektierbar ist und detektierte ranzige Früchte einzeln in Sortieranlagen aus dem Produktstrom trennbar sind.

In einer bevorzugten Ausführungsform der Erfindung umfasst das Identifizieren der flüchtigen Komponenten der Probe durch massenspektroskopische Detektion von für die Lipidoxidation relevanten Bestandteilen das Identifizieren von einer oder mehr Stoffgruppen/Funktionsgruppen, ausgewählt aus:
- Hydroperoxide
- Zyklische Hydroperoxide
- Gesättigte, einfach und zweifach ungesättigte Aldehyde
- Kohlenwasserstoffe (Alkane, Alkene)
- Alkohole (gesättigt und ungesättigt)
- Ketone (gesättigt und ungesättigt)
- Kurzkettige Fettsäuren
- Alkylfurane.

Insbesondere kann das Identifizieren der flüchtigen Komponenten der Probe durch massenspektroskopische Detektion von für die Lipidoxidation relevanten Bestandteilen das Erstellen von Chromatogrammen bei in einem Bereich zwischen 20 und 300 ausgewählten Masse/Ladungsverhältnissen erfolgen, vorzugsweise bei zumindest einem Masse/Ladungsverhältnis, ausgewählt aus 43, 44, 55, 56, 57, 60, 70, 71, 73, 74, 81, 83, 97.

In einer spezifischen Ausführungsform der Erfindung umfasst das Identifizieren der flüchtigen Komponenten der Probe durch massenspektroskopische Detektion von für die Lipidoxidation relevanten Bestandteilen das Erstellen eines Fettsäurenchromatogramms bei einem ersten Masse/Ladungsverhältnis, sowie eines Aldehydchromatogramms bei einem zweiten Masse/Ladungsverhältnis.

Die Proben, mit denen das erfindungsgemäße Verfahren durchgeführt, sind in Zahl und Eigenschaft solcherart ausgewählt, dass ein repräsentativer Querschnitt an möglichen Ranzigkeiten ermittelt werden kann. Dies kann unter anderem durch eine geeignete Probenauswahl und -vorbereitung (z.B. Lagerung bei erhöhter Temperatur für verschiedene Zeitperioden) sichergestellt werden.

Bei der von der Erfindung bevorzugten Messung der Absorptions- oder Reflektionsspektren der Ölfrüchte, Nüsse bzw. Samen durch Hyper-Spektral-Bildgebung (HSI) zur Bestimmung der Ranzigkeit und anschließende Sortierung sind detaillierte Informationen der einzelnen Ölfrüchte, Nüsse bzw. Samen für ein gutes Kalibriermodell wesentlich. Dies wird durch das erfindungsgemäße Verfahren gewährleistet, wodurch eine wesentlich zielsicherere Aussortierung von ranzigen Ölfrüchten, Nüssen bzw. Samen erzielt werden kann als bisher. Insbesondere bietet die Erfindung eine hochgenaue und unterscheidungskräftige Referenzanalytik, mit der der tatsächliche Oxidationsstatus einzelner Nüsse/Ölfrüchte/Samen bestimmt und Extremwerte davon erfasst werden können.

Eine Ausführungsform des erfindungsgemäßen Verfahrens basiert auf der Anreicherung flüchtiger Verbindungen im Dampfraum über der Probe, im speziellen auf Headspace Solid Phase Microextraction (HS-SPME), gekoppelt an einen Gaschromatographen mit massenselektiver Detektion. Diese Kombination von Analyseverfahren wird mit HS-SPME-GC-MS abgekürzt. Die Erfindung ist jedoch nicht auf diese Ausführungsform beschränkt, sondern das erfindungsgemäße Verfahren umfasst grundsätzlich die Messung und Bestimmung flüchtiger Verbindungen im Dampfraum der Probe, das Trennen einzelner Bestandteile der flüchtigen Verbindungen mittels Gaschromatographie, das Identifizieren durch massenselektive Detektieren zumindest einzelner der getrennten flüchtigen Verbindungen und das selektive Heranziehen von identifizierten flüchtigen Verbindungen zur Bestimmung des Ranzigkeitsindex.

Die Lipidoxidation von ungesättigten Fettsäuren beginnt mit der Bildung von Hydroperoxiden und führt zu einer großen Gruppe von verschiedenen chemischen Strukturen und funktionellen Gruppen. So können diese Stoffgruppen potentiell als Markerverbindungen verwendet werden, um den Grad der Lipidoxidation zu messen.

Es empfiehlt sich für die Hyperspektralanalyse alle Wellenlängen im nahen Infrarotbereich zu berücksichtigen, die die strukturellen Eigenschaften, die oben beschrieben wurden, messen können.

Verbindungen, die für einen unerwünschten ranzigen Geschmack verantwortlich sind, sind beispielsweise Aldehyde, die sich nach der Hydroperoxidbildung von der Fettsäurekettenspaltung ableiten. Bei fortlaufender Oxidation können die Aldehyde freie Fettsäuren bilden, die auch zu weiteren unerwünschten sensorischen Eigenschaften von ranzigen Nüssen beitragen können. Für die Bewertung der Qualität einzelner Ölfrüchte oder Samen werden diese (mit unterschiedlicher Ranzigkeit und unterschiedlicher Herkunft) zuerst durch HSI gemessen, zeitnah einzeln markiert und verpackt und von HS-SPME-GC-MS wie folgt analysiert:
Einzelne Ölfrüchte, Nüsse oder Samen werden gemahlen und eine geeignete repräsentative Probenmenge, z.B. 300 mg, wird in ein Glasgefäß geeigneter Größe eingewogen und gasdicht verschlossen. Es kann ein glasbeschichteter Magnetrührer im Glasgefäß enthalten sein. Es erfolgt eine Anreicherung der flüchtigen Komponenten mit geeigneten Techniken basierend auf Dampfraumanalyse an geeigneten ad- oder absorptiven Materialien, die in der Lage sind, flüchtige organische Verbindungen reversibel zu binden. Weiters erfolgt eine Desorption thermisch bei erhöhten Temperaturen, vorzugsweise direkt im Einlasssystem eines gaschromatographischen Systems. Die Trennung der flüchtigen Verbindungen erfolgt auf hochauflösenden Kapillarsäulen mit einer geeigneten stationären Phase und einem Temperaturprogramm, welches in der Lage ist, die Analyten zu trennen. Die Detektion erfolgt mittels massenselektiver Detektion in der Art und Weise, dass ein Massenspektrum über den gesamten Massenbereich der relevanten Zielverbindungen erfasst werden kann, um eine eindeutige Identifikation der Verbindungen zu ermöglichen Massenspektren werden im ScanModus mit einem Scan-Bereich von Masse/Ladungsverhältnissen (m/z) von vorzugsweise 20-300 erfasst.

Weitere Informationen können aus dem Extrahieren von ausgewählten Masse/Ladungsverhältnissen gewonnen werden. Es hat sich gezeigt, dass m/z = 44 ein universelles und ausgewähltes Fragment für lineare und gesättigte Aldehyde darstellt, dass für die Bestimmung der Ranzigkeit hervorragend geeignet ist. Zusätzliche Information über potentielle Ranzigkeit können über freie Fettsäure unter Verwendung des Masse/Ladungsverhältniss m/z = 60 erhalten. Gemäß einer Ausführungsform des Verfahrens wird in Folge dessen das Aldehydchromatogramm bei einem m/z von 44 und das Fettsäurenchromatogramm bei einem m/z von 60 erstellt.

In einer Ausführungsform der Erfindung kann ein Ranzigkeitsindexwert durch die Integration der Peaks des Aldehydchromatogramms mit einem m/z von 44 und des Fettsäurenchromatogramms mit einem m/z von 60 berechnet werden. Die erhaltenen Zahlen können entweder als Aldehydindexwert oder als Fettsäureindexwert oder als Totalranzigkeitsindex ausgedrückt werden. Für eine bessere Lesbarkeit wird die Summe der Peak-Bereiche durch eine feste Zahl geteilt, um eine Zahl zu erhalten, die bequemer zu handhaben ist. Durch die Analyse einer großen Gruppe von verschiedenen Proben unterschiedlicher Herkunft und Qualität kann eine breite Palette von Ranzigkeitsindexwerten ermittelt bzw. überprüft werden, die für das Kalibrierungsmodell verwendet werden.

Das erfindungsgemäße Verfahren verwendet somit einen quantitativen Ansatz, bei dem nicht nach offensichtlichen Unterscheidungsmerkmalen in den Absorptions- oder Reflektionsspektren gesucht wird, sondern schwache, aber noch signifikante Unterschiede in den Absorptions- oder Reflektionsspektren in Korrelation zu einer Referenz aus dem Labor gestellt wird. Die Absorptions- oder Reflektionsspektren werden somit nicht dazu verwendet, Ölfrüchte und Samen nach "Gut" (kaum bis keine Produkte einer Ranzigkeitsreaktion) und "Schlecht" (Produkte einer Ranzigkeitsreaktion vorhanden) in zwei Kategorien zu trennen, sondern es wird ein Ranzigkeitsindex erstellt. Hierdurch wird der Vorteil erreicht, dass somit quantitativ der Grad der Ranzigkeit erfasst werden kann. Besonders vorteilhaft ist, dass erst in einem nachfolgenden Schritt ein oder mehrere Schwellenwerte für den Ranzigkeitsindex bereitgestellt werden kann/können, ab welchem eine Ölfrucht, eine Nuss beziehungsweise ein Samen als nicht mehr qualitätskonform bzw. in unterschiedliche Qualitätsstufen fallend eingestuft wird. Dies ermöglicht insbesondere den Vorteil einer sehr einfachen Anpassung an unterschiedliche Qualitätsansprüche für Ölfrüchte und Samen.

Das erfindungsgemäße Verfahren bietet des Weiteren den Vorteil, dass erstmals eine Identifizierbarkeit der Ranzigkeit von Ölfrüchten und Samen mittels Spektrometrie ermöglicht wird. Hierzu wurden im Labor chemische Marker gefunden, die direkt mit der Ranzigkeit korrelieren. Danach wurden erneut mit Hilfe von statistischen Korrelationsmethoden die an ausgewählten Proben aufgenommenen Absorptions- oder Reflektionsspektren des Fotosensors mit den für dieselben Proben im Labor ermittelten Ranzigkeitsindex korreliert, sodass aus dem Absorptions- oder Reflektionsspektrum direkt der Ranzigkeitsindex errechnet werden kann.

Vorteilhafte Ausgestaltungen des erfindungsgemäßen Verfahrens, sowie alternativer Ausführungsvarianten werden in weiterer Folge, und anhand der Figuren näher erläutert.
Figur 1 zeigt ein Chromatogramm einer ranzigen Probe und einer frischen Probe.
Figur 2 zeigt ein Chromatogramm der extrahierten m/z 44 (gesättigte Aldehyde) einer ranzigen und einer frischen Nuss.
Figur 3 zeigt Chromatogramme der extrahierten m/z 60 (Fettsäuren) einer ranzigen und einer frischen Nuss.
Figur 4 zeigt eine Vorrichtung zur Detektion ranziger Ölfrüchte, Nüsse oder Samen in einer schematischen Darstellung.

Das erfindungsgemäße Verfahren stellt eine Zuordnung eines Ranzigkeitsindexwert zu einzelnen Ölfrüchten, Nüssen und Samen 2 bereit, wobei in einem ersten Verfahrensschritt eine einzelne Ölfrucht, Nuss oder ein Samen 2 mit einer Lichtquelle 3 bestrahlt wird. Dies erfolgt gemäß einer bevorzugten Ausführungsvariante im Nahinfrarotbereich.

Das von der Ölfrucht, Nuss oder dem Samen 2 reflektierte oder durch diese bzw. diesen transmittierte Licht wird in weiterer Folge auf einen Fotosensor 4 projiziert, welcher ein Absorptions- oder Reflektionsspektrum in einem Nahinfrarotbereich von 900 bis 2500 nm, vorzugsweise 900 bis 1700 nm erfasst. In einer besonders bevorzugten Ausführungsvariante wird das Absorptions- oder Reflektionsspektrum in einem Bereich von 1000 bis 1500nm durch den Fotosensor 4 erfasst. In einer bevorzugten Ausführungsvariante erfolgt das Erfassen des Absorptions- oder Reflektionsspektrum durch hyperspektrale Erfassung.

Zur Bestimmung des Ranzigkeitsindexwerts der Ölfrucht, Nuss oder des Samens 2, dessen Absorptions- oder Reflektionsspektrum zuvor erfasst wurde, wird zeitnah im Anschluss an die Messung des Spektrums die flüchtige Fraktion durch Dampfraumanalyse der flüchtigen Fraktion einzelner homogenisierter Ölfrüchte/Samen/Nüsse an geeigneten ad- und/oder absorptiven Materialien angereicht. Nach thermischer Desorption erfolgt die Trennung und Detektion an einem gaschromatographischen System mit massenselektiver Detektion. Die Auswahl entsprechender selektiver Massenfragmente der durch die Lipidoxidation gebildeten Abbauprodukte ermöglicht eine eindeutige Zuordnung zu relevanten Substanzklassen und damit zum Erstellen eines geeigneten Kalibrationsmodells für die spektralen Daten aus der Spektrumsmessung, insbesondere HSI Messung.

In einer bevorzugten Ausführungsvariante erfolgt das Bestimmen eines Aldehydindexwerts durch Integrieren über zumindest einen Bereich eines ermittelten Aldehydchromatogramms, und das Bestimmen eines Fettsäureindexwerts durch Integrieren über zumindest einen Bereich eines ermittelten Fettsäurenchromatogramms.

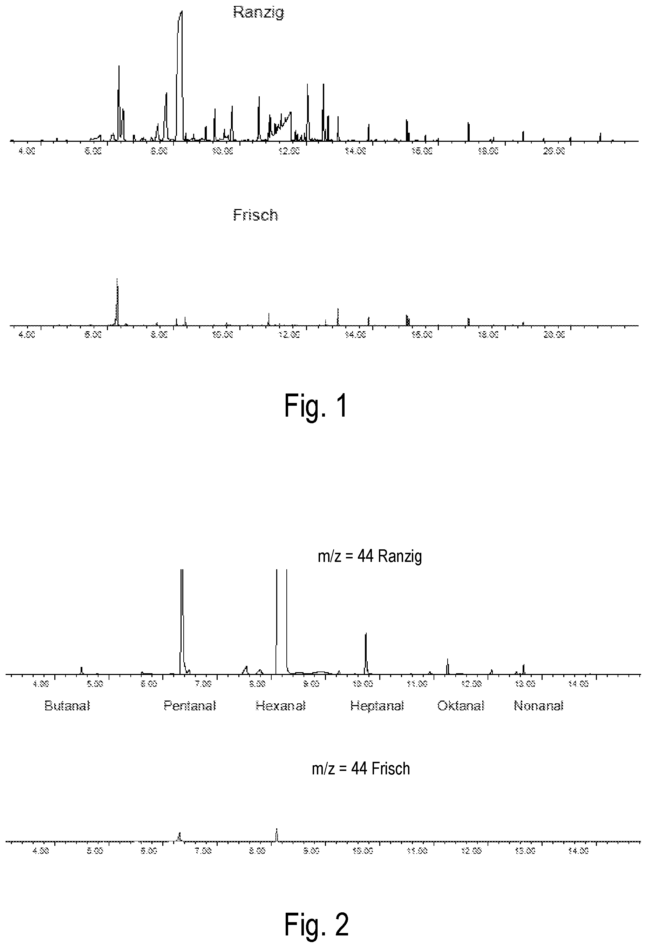

Figur 1 zeigt zur Veranschaulichung ein Chromatogramm einer ranzigen Probe und einer frischen Probe welche gemäß dem zuvor beschriebenen Verfahren analysiert wurden, wobei das Massenspektrometer bei der Erfassung in einem Scanmodus von Masse/Ladungsverhältnissen (m/z) in einem relevanten Massenbereich, z.B. von 20-300, betrieben wurde.

Figur 2 zeigt zur Veranschaulichung ein Beispiel eines Aldehydchromatogramms einer ranzigen Probe bei dem ersten Masse/Ladungsverhältnis (m/z) von 44 im Vergleich zu einer frischen Probe. Die wie oben beschriebene Bestimmung des Aldehydindexwerts führt für das Aldehydchromatogramm der ranzigen Probe offensichtlich zu einem wesentlich höheren Aldehydindexwert als für die gute Probe.

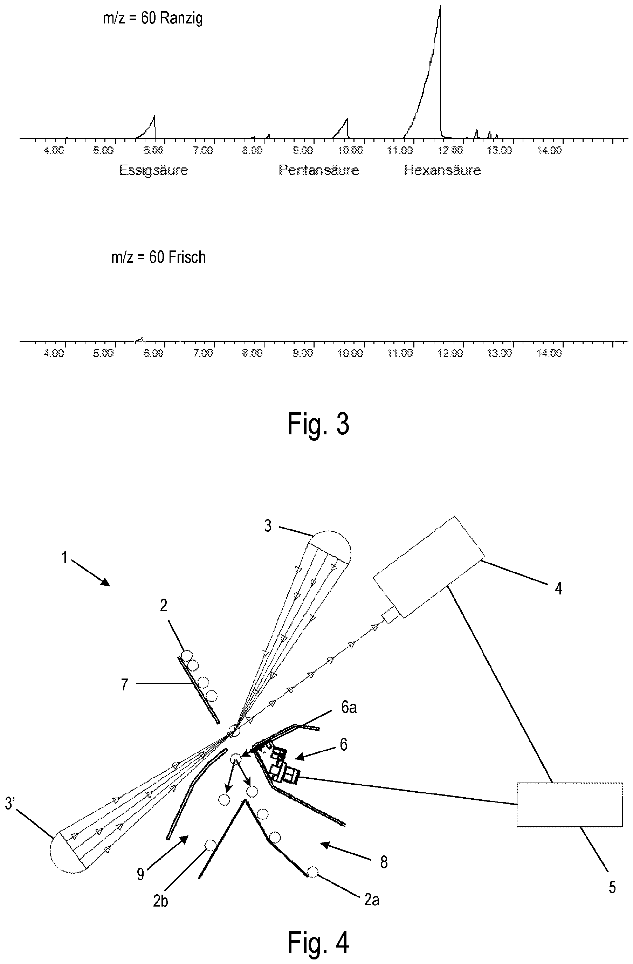

Figur 3 zeigt zur Veranschaulichung ein Beispiel eines Fettsäurechromatogramms einer ranzigen Probe bei dem zweiten Masse/Ladungsverhältnis (m/z) von 60 im Vergleich zu einer frischen Probe. Die wie oben beschriebene Bestimmung des Fettsäureindexwerts führt für das Fettsäurechromatogramm der ranzigen Probe offensichtlich zu einem wesentlich höheren Fettsäureindexwert als für die frische Probe analog zum in Figur 2 beschriebenen Aldehydindexwert.

Diese Schritte werden für eine repräsentative Vielzahl an Ölfrüchten, Nüssen beziehungsweise Samen wiederholt, und eine Ranzigkeitsindextabelle aus den ermittelten Ranzigkeitsindexwerten und den zugeordneten Absorptions- und Reflektionsspektren erstellt.

In einer bevorzugten Ausführungsvariante des Verfahrens erfolgt das Zuordnen des erfassten Absorptions- oder Reflektionsspektrums der Ölfrucht, Nuss oder des Samens zu dem Ranzigkeitsindexwert durch Zuordnung des Ranzigkeitsindexwerts zu zumindest einem aus einem Mittelwert, einer Bandbreite oder einzelner Frequenzbänder des erfassten Absorptions- oder Reflektionsspektrums. Hierbei werden bestimmte Bereiche oder ein Mittelwert des jeweiligen Absorptions- oder Reflektionsspektrums als Bereiche festgelegt, welche charakteristisch für den Grad der Ranzigkeit der Ölfrüchte, Nüsse oder Samen 2 sind.

Des Weiteren stellt die Erfindung zur Lösung der eingangs gestellten Aufgaben ein Verfahren zur Detektion der Ranzigkeit einer Ölfrucht, Nuss oder eines Samens 2 bereit. In diesem Detektionsverfahren wird in einem ersten Verfahrensschritt eine einzelne Ölfrucht, Nuss oder ein Samen mit einer Lichtquelle bestrahlt. Dies erfolgt gemäß einer bevorzugten Ausführungsvariante ebenfalls im Nahinfrarotbereich.

Das von der Ölfrucht, Nuss oder dem Samen reflektierte oder durch diese bzw. diesen hindurch transmittierte Licht wird in weiterer Folge auf einen Fotosensor projiziert, welcher ein Absorptions- oder Reflektionsspektrum in einem Nahinfrarotbereich von vorzugsweise 900 bis 1700 nm erfasst. In einer besonders bevorzugten Ausführungsvariante wird das Absorptions- oder Reflektionsspektrum in einem Bereich von 1000 bis 1500 nm durch den Fotosensor erfasst. Bevorzugt erfolgt das Erfassen des Absorptions- oder Reflektionsspektrums durch hyperspektrale Erfassung mittels einer Hyperspektralkamera.

In einem weiteren Schritt greift dieses Verfahren auf die Ranzigkeitstabelle des zuvor beschriebenen Verfahrens zurück, welche die Ranzigkeitsindexwerte und die zugeordneten Absorptions- oder Reflektionsspektren bzw. charakteristische Bereiche und/oder Wellenlängen dieser Spektren enthält. Es folgt ein Vergleich des in diesem Verfahren erfassten Absorptions- oder Reflektionsspektrums mit den Absorptions- oder Reflektionsspektren, welche in der Ranzigkeitsindextabelle enthalten sind. Hierdurch wird eine Zuordnung des erfassten Absorptions- oder Reflektionsspektrums zu einem dem erfassten Absorptions- oder Reflektionsspektrum ähnlichsten Absorptions- oder Reflektionsspektrum der Ranzigkeitsindextabelle getroffen. Dies ermöglicht das Ermitteln des dem ähnlichsten Absorptions- oder Reflektionsspektrum zugeordneten Ranzigkeitsindexwerts.

Dieses Verfahren bietet den Vorteil, dass durch eine vorangegangene Kalibrierung mittels des zuvor beschriebenen erfindungsgemäßen Verfahrens zur Erstellung einer Ranzigkeitsindextabelle die Detektion der Ranzigkeit einer Ölfrucht, Nuss oder eines Samens in einer Produktionsanlage ermöglicht wird, wobei die Ranzigkeit einzelner Früchte detektiert wird und nicht nur ein homogener Produktstrom.

Gemäß einer bevorzugten Ausführungsvariante des erfindungsgemäßen Detektionsverfahrens erfolgt die Zuordnung des erfassten Absorptions- oder Reflektionsspektrums zu einem dem erfassten Absorptions- oder Reflektionsspektrum ähnlichsten Absorptions- oder Reflektionsspektrum der Ranzigkeitsindextabelle durch Vergleich von zumindest einem aus einem Mittelwert, einer Bandbreite oder einzelner Frequenzbänder der Absorptions- oder Reflektionsspektren. Hierbei werden bestimmte Bereiche, oder ein Mittelwert des jeweiligen Absorptions- oder Reflektionsspektrums als Bereiche festgelegt, welche charakteristisch für den Grad der Ranzigkeit der Ölfrüchte, Nüsse oder Samen sind und zu diesem Vergleich herangezogen. Dies erhöht vorteilhafterweise die Genauigkeit des Verfahrens.

Des Weiteren bietet dieses Detektionsverfahren den Vorteil, dass in einem weiteren Verfahrensschritt zumindest ein Schwellenwert festgelegt werden kann und ein Überschreiten dieses zumindest einen Schwellenwerts das Ausscheiden oder differenzierte Sortieren der Ölfrucht, Nuss oder des Samens bewirkt. Dies bietet den Vorteil, dass durch die Festlegung eines Schwellenwerts eine Anpassung an verschiedene Qualitätsanforderungen für die Ölfrüchte, Nüsse bzw. die Samen ermöglicht wird.

Schließlich umfasst die Erfindung auch eine nachfolgend detailliert beschriebene Vorrichtung zur Detektion ranziger Ölfrüchte, Nüsse oder Samen, wobei diese Vorrichtung dazu ausgebildet ist, das erfindungsgemäße Detektionsverfahren abzuarbeiten, wobei eine gemäß dem erfindungsgemäßen Verfahren zur Erstellung einer Ranzigkeitsindextabelle erstellte Ranzigkeitsindextabelle verwendet wird.

Figur 4 zeigt eine erfindungsgemäße Vorrichtung 1 zur Detektion ranziger Ölfrüchte, Nüsse oder Samen 2 in einer schematischen Darstellung, welche zwei Lichtquellen 3, 3', die gemeinsam oder alternativ vorgesehen sein können, einen Fotosensor 4, eine Rechnereinheit 5 und eine Sortiereinheit 6 umfasst. Die Ölfrüchte, Nüsse oder Samen 2 werden mittels einer Transportvorrichtung 7 in Form einer Rutsche nacheinander durch die von der Lichtquelle 3, 3a emittierten Lichtstrahlen vorbeigeführt und von diesen bestrahlt. Gemäß einer bevorzugten Ausführungsvariante emittieren die Lichtquellen 3, 3' Licht im Nahinfrarotbereich. Der Fotosensor 4 detektiert das von der Ölfrucht, der Nuss bzw. dem Samen 2 reflektierte Licht der Lichtquelle 3 oder transmittierte Licht der Lichtquelle 3' und erfasst das Absorptions- oder Reflexionsspektrum der Ölfrucht, Nuss oder des Samens 2.

Der Fotosensor 4 ist mit der Rechnereinheit 5 verbunden und übermittelt an diese das erfasste Absorptions- oder Reflexionsspektrum.

Die Rechnereinheit 5 greift zur Bewertung des Absorptions- oder Reflexionsspektrums auf die Ranzigkeitsindextabelle gemäß dem hierin zuerst beschriebenen Verfahren zurück. Diese enthält eine Reihe an Ranzigkeitsindexwerten und zugeordneten Absorptions- oder Reflektionsspektren. Die Rechnereinheit 5 vergleicht das erfasste Absorptions- oder Reflexionsspektrum mit den in der Ranzigkeitsindextabelle angeführten Absorptions- oder Reflexionsspektren und ordnet das erfasste Absorptions- oder Reflektionsspektrum dem ähnlichsten Absorptions- oder Reflektionsspektrum der Ranzigkeitsindextabelle zu. Dies erfolgt gemäß einer bevorzugten Ausführungsvariante durch einen Vergleich von einem Mittelwert, einer Bandbreite oder einzelner Frequenzbänder der Absorptions- oder Reflektionsspektren, oder einer Kombination davon. Nach erfolgter Zuordnung ermittelt die Rechnereinheit den dem ähnlichsten Absorptions- oder Reflektionsspektrum zugeordneten Ranzigkeitsindexwert. Hierdurch wird jeder einzelnen durch die Vorrichtung 1 detektierten Ölfrucht, Nuss bzw. Samen 2 ein Ranzigkeitsindexwert zugeordnet.

Nachgeordnet zum Fotosensor 4 werden die Ölfrüchte, Nüsse oder Samen 1 an der Sortiereinheit 6 vorbeigeführt, welche ebenfalls mit der Rechnereinheit 5 verbunden ist, wobei die Rechnereinheit 5 die Sortiereinheit 6 steuert. Die Sortiereinheit 6 ermöglicht eine Sortierung einzelner Ölfrüchte, Nüsse oder Samen 2 aus einem Produktstrom von durch die Vorrichtung 1 geführten Ölfrüchten, Nüssen oder Samen 2, beispielsweise mittels Druckluftstößen 6a, die die Ölfrüchte, Nüsse oder Samen je nach zugeordnetem Ranzigkeitsindexwert in unterschiedliche Sortiergänge 8 für Gutprodukte 2a und 9 für ranzige Produkte 2b befördern, wobei die Sortiergänge 8, 9 z.B. durch Rutschen realisiert sind. Die Rechnereinheit 5 trifft anhand eines vorgegebenen Schwellenwerts für den Ranzigkeitsindexwert eine Entscheidung die jeweilige Ölfrucht, Nuss bzw. den jeweiligen Samen 2 in einen der Sortiergänge zu befördern. Die Sortiereinheit 6 kann als Klappenvorrichtung, Druckluftvorrichtung (wie dargestellt) oder ähnliches ausgeführt sein. Weitere Ausführungsformen der Sortiereinheit 6 ergeben sich für den Fachmann aus diesem beispielhaften Verweis.

Zusammenfassend liegen der vorliegenden Erfindung, wie in den unabhängigen Ansprüchen und den bevorzugten Ausführungsformen definiert, die folgenden Konzepte und Vorteile zugrunde:
Die Detektion und Sortierung von Schüttgütern mithilfe von Fotosensoren mittels Hyperspectral Imaging (HSI) ist eine gängige Methode. Dabei wird eine Probe mit breitbandigem Licht bestrahlt und das reflektierte Licht von einem Fotosensor, vorzugsweise im nahen Infrarotbereich, erfasst und spektroskopisch untersucht. Auf Grund der Auswertung der Spektren (Amplitude, Frequenz) wird auf Eigenschaften bzw. Inhaltsstoffe geschlossen. Der Nachweis dieser Eigenschaften, Inhaltsstoffe ist die Basis für die Unterscheidung von guten und schlechten Produkten im Sortierprozess. Der Nachweis dieser Eigenschaften/Inhaltsstoffe basiert auf einem qualitativen, relativen Ansatz. Es werden in einem Modellierprozess die photometrisch erfassten Spektrenverläufe der Gutprodukte mit dem Schlechtprodukt verglichen. Dabei werden Bereiche des Spektrums gesucht, in denen der Unterschied der Eigenschaften sehr groß ist bzw. die Korrelation zu einer gesuchten Substanz im Unterschied zu anderen Substanzen sehr hoch ist. Der/die identifizierten Bereich(e) werden dann für den Sortierprozess ausgewählt und die jeweiligen Spektren normiert. Das heißt, dass die absoluten Amplituden des Spektrums eliminiert und nur die Unterschiede zwischen dem Spektrum des Gut- und Schlechtproduktes für die Entscheidung in der Sortierung herangezogen werden.

Bedingt durch diese Vorgangsweise kommt es aus folgenden Gründen zu Fehlklas sifikationen:
- Sind die Unterschiede zwischen den beiden Spektren sehr klein, so wird die kleine Differenz im Normierungsprozess derart stark vergrößert, dass das Signal/Rauschverhältnis stark steigt, wodurch sich die Unsicherheit der Entscheidung stark erhöht.
- Die Unterschiede im Spektrum werden anhand einer Referenzprobe von guten/schlechten Exemplaren bestimmt. Soweit es sich aber um Naturprodukte mit spektraler Streuung handelt, ist dieser Vergleich mit einer hohen Unsicherheit behaftet und kann bei jeder Produktcharge schwanken.

Das Resultat dieser Fehlklassifikation ist eine mangelhafte Trennung von guten und verdorbenen Lebensmittelen.

Dieses Problem tritt insbesondere bei Ölfrüchten, Nüssen und Samen auf, wenn sie in Produktionsanlagen automatisiert verarbeitet werden. Bei dieser automatisierten Verarbeitung steht das Interesse ranzige und somit verdorbene Elemente von nicht ranzigen Elementen automatisiert zu unterschieden, im Mittelpunkt. Noch bevor die Produkte weiterverarbeitet werden (Pressen, Reiben, Schälen, etc.), sollen einzelne, qualitativ minderwertige Früchte/Nüsse/Samen automatisiert, mit hoher Durchsatzrate, eliminiert werden.

Die vorgestellte Lösung basiert auf einem quantitativen Ansatz, der die Nachteile der bisherigen Verfahren beseitigt, indem nicht wie bisher, die Sortierinformation aus dem spektralen Vergleich von ranzigen Ölfrüchte/Nüsse/Samen mit nicht ranzigen Vergleichsmengen gewonnen wird, sondern die chemischen Auslöser der Ranzigkeit (Lipidoxidation, Hydrolyse) und die dabei entstehenden Stoffe (Aldehyde, etc.) hinsichtlich ihres spektralen Fingerabdrucks untersucht werden.

Da die Ranzigkeit keine zweiwertige Größe ist, d.h. eine bloße Unterscheidung in ranzig/nicht ranzig unzureichend ist, sondern Ranzigkeit in verschiedenen Graden vorliegt, wird eine erfindungsgemäß entwickelte Ranzigkeitsindextabelle verwendet, welche die Amplituden im entsprechenden spektralen Bereich einem Ranzigkeitswert von z.B. 0-100% zuordnet.

Diese Ranzigkeitsindextabelle wird unter Verwendung der zur Verfügung stehenden analytischen Labormethoden (z.B. Gaschromatographie etc.) anhand einer statistisch großen Menge von Ölfrüchten/Nüssen/Samen entwickelt.

Die Ranzigkeitsindextabelle wird nun erfindungsgemäß unter Anwendung eines Fotosensors einer Sortieranlage, bevorzugt einer Hyperspektralkamera, zur Detektion des Ranzigkeitsgrads von Ölfrüchten, Nüssen und Samen verwendet, wobei der Fotosensor mithilfe der Ranzigkeitsindextabelle der Sortieranlage kalibriert werden kann. Das heißt, dass im maßgebenden Spektralbereich anhand dieser Tabelle von der absoluten Amplitude im Spektrum automatisch auf den quantitativen Grad (z.B. 0-100%) der Ranzigkeit geschlossen werden kann.

Das auf der erfindungsgemäß erstellten Ranzigkeitsindextabelle basierende Detektionsverfahren bzw. eine dieses Detektionsverfahren abarbeitende Sortieranlage zeichnet sich durch folgende Vorteile aus:
- Hohe Verarbeitungsgeschwindigkeit und Entscheidungssicherheit im online Sortierprozess, bedingt durch die Auswertung der Ranzigkeitsindextabelle;
- Qualitativ hochwertige Ranzigkeitsindextabelle, basierend auf dem aktuellen Stand der offline Labortechnik, kann online im Sortierprozess verwendet werden;
- Die Sortierung der Ranzigkeit ist keine zweiwertige Größe (ranzig/nicht ranzig), sondern eine analoge Größe, die basierend auf der Amplitude bei einer gewissen Wellenlänge bzw. auf den Mittelwerten der Amplituden in einem Wellenlängenbereich zurückgeführt werden kann;
- Basierend auf einem Ranzigkeitswert kann eine Sortierung nach verschiedenen Qualitäten (x % Ranzigkeit) durchgeführt werden und somit eine Verwertung von verschiedenen Qualitätsstufen erzielt werden;
- Exakte Einstellung des Sortiergrenzwertes in der Sortieranlage.

## Patentansprüche

1. Verfahren zum Erstellen einer Ranzigkeitsindextabelle und Zuordnen eines Ranzigkeitsindexwerts zu einem Absorptions- oder Reflektionsspektrums von Ölfrüchten, Nüssen und Samen (2), umfassend die Schritte:
- Bestrahlen einer Probe einer Ölfrucht, Nuss oder eines Samens (2) mit einer Lichtquelle (3),
- Projizieren des reflektierten und/oder transmittierten Lichts auf einen Fotosensor (4),
- Erfassen des Absorptions- oder Reflektionsspektrums in einem Wellenlängenbereich von 900 bis 2500 nm, vorzugsweise 900 bis 1700 nm, noch bevorzugter 1000 bis 1500 nm, durch den Fotosensor (4),
- Extrahieren von Inhaltsstoffen der Probe durch Probenvorbereitungstechniken auf Basis einer Bestimmung flüchtiger Verbindungen aus einem Dampfraum über der Probe, vorzugsweise Festphasenmikroextraktion,
- Trennen von flüchtigen Komponenten der Probe mittels gaschromatographischer Techniken,
- Identifizieren von abgetrennten flüchtigen Komponenten der Probe durch massenspektroskopische Detektion von für die Lipidoxidation relevanten Bestandteilen der flüchtigen Komponenten,
**dadurch gekennzeichnet, dass** das Verfahren die Schritte
- Bestimmen eines Ranzigkeitsindexwerts der Probe aus identifizierten flüchtigen Komponenten der Probe,
- Zuordnen zumindest von einzelnen charakteristischen Wellenlängen oder Wellenlängenbereichen des erfassten Absorptions- oder Reflektionsspektrums der Probe zu dem Ranzigkeitsindexwert,
- Wiederholen der vorangegangenen Schritte für eine repräsentative Anzahl an Proben und Bilden einer Ranzigkeitstabelle aus den ermittelten Ranzigkeitsindexwerten und zugeordneten Absorptions- oder Reflektionsspektren bzw. charakteristischen Wellenlängen oder Wellenlängenbereichen der erfassten Absorptions- oder Reflektionsspektren
umfasst.

2. Verfahren gemäß Anspruch 1, **dadurch gekennzeichnet, dass** das Identifizieren der flüchtigen Komponenten der Probe durch massenspektroskopische Detektion von für die Lipidoxidation relevanten Bestandteilen das Identifizieren von einer oder mehreren Stoffgruppen/Funktionsgruppen, ausgewählt aus:
- Hydroperoxide
- Zyklische Hydroperoxide
- Gesättigte, einfach und zweifach ungesättigte Aldehyde.
- Kohlenwasserstoffe (Alkane, Alkene)
- Alkohole (gesättigt und ungesättigt)
- Ketone (gesättigt und ungesättigt)
- Kurzkettige Fettsäuren
- Alkylfurane
umfasst.

3. Verfahren gemäß Anspruch 1 oder 2, **dadurch gekennzeichnet, dass** das Identifizieren der flüchtigen Komponenten der Probe durch massenspektroskopische Detektion von für die Lipidoxidation relevanten Bestandteilen das Erstellen von Chromatogrammen bei in einem Bereich zwischen 20 und 300 ausgewählten Masse/Ladungsverhältnissen erfolgt, vorzugsweise bei zumindest einem Masse/Ladungsverhältnis, ausgewählt aus 43, 44,55,56,57, 60, 70, 71, 73, 74, 81, 83, 97.

4. Verfahren nach Anspruch 3, **gekennzeichnet durch** das Erstellen eines Fettsäurenchromatogramms bei einem für Fettsäuren charakteristischen Masse/Ladungsverhältnis, insbesondere bei einem Masse/Ladungsverhältnis von 60.

5. Verfahren gemäß Anspruch 4, **gekennzeichnet durch** das Bestimmen eines Fettsäureindexwerts durch Integrieren über zumindest einen Bereich des Fettsäurenchromatogramms.

6. Verfahren nach einem der Ansprüche 3 bis 5, **gekennzeichnet durch** das Erstellen eines Aldehydchromatogramms bei einem für Aldehyde charakteristischen Masse/Ladungsverhältnis, insbesondere bei einem Masse/Ladungsverhältnis von 44.

7. Verfahren gemäß Anspruch 6, **gekennzeichnet durch** das Bestimmen eines Aldehyhydindexwerts durch Integrieren über zumindest einen Bereich des Aldehydchromatogramms.

8. Verfahren gemäß den Ansprüchen 5 und 7, **dadurch gekennzeichnet, dass** das Bestimmen eines Ranzigkeitsindexwerts durch Bildung der Summe des Aldehydindexwerts und des Fettsäureindexwerts erfolgt.

9. Verfahren nach einem der vorhergehenden Ansprüche, **dadurch gekennzeichnet, dass** das Zuordnen der erfassten Absorptions- oder Reflektionsspektren der Probenzu den Ranzigkeitsindexwerten durch Zuordnung der Ranzigkeitsindexwerten zu zumindest einem aus einem Mittelwert, einer Bandbreite oder einzelner Frequenzbänder der erfassten Absorptions- oder Reflektionsspektren erfolgt.

10. Verfahren gemäß einem der vorhergehenden Ansprüche, **dadurch gekennzeichnet, dass** das Erfassen des Absorptions- oder Reflektionsspektrums durch den Fotosensor (4) durch hyperspektrale Erfassung erfolgt.

11. Verfahren zur Detektion der Ranzigkeit einer Ölfrucht, Nuss oder eines Samens (2), umfassend die Schritte:
- Bestrahlen der Ölfrucht, Nuss oder des Samens (2) mit zumindest einer Lichtquelle (3),
- Projizieren des reflektierten und/oder transmittierten Lichts auf einen Fotosensor (4),
- Erfassen eines Absorptions- oder Reflektionsspektrums in einem Wellenlängenbereich von 900 bis 2500 nm, vorzugsweise 900 bis 1700 nm, noch bevorzugter 1000 bis 1500 nm, durch den Fotosensor (4),
**dadurch gekennzeichnet, dass** das Verfahren die Schritte
- Bereitstellen einer Ranzigkeitsindextabelle gemäß einem der Ansprüche 1 bis 10, welche die Ranzigkeitsindexwerte und die zugeordneten Absorptions- oder Reflektionsspektren bzw. charakteristischen Wellenlängen oder Wellenlängenbereichen des Absorptions- oder Reflektionsspektrums enthält,
- Zuordnen des erfassten Absorptions- oder Reflektionsspektrums bzw.
charakteristischen Wellenlängen oder Wellenlängenbereichen des erfassten Absorptions- oder Reflektionsspektrums zu einem dem erfassten Absorptions- oder Reflektionsspektrum ähnlichsten Absorptions- oder Reflektionsspektrum der Ranzigkeitsindextabelle bzw. charakteristischen Wellenlängen oder Wellenlängenbereichen des Absorptions- oder Reflektionsspektrums,
- Ermitteln des dem ähnlichsten Absorptions- oder Reflektionsspektrum bzw. charakteristischen Wellenlängen oder Wellenlängenbereichen des Absorptions- oder Reflektionsspektrums zugeordneten Ranzigkeitsindexwerts
umfasst.

12. Verfahren gemäß Anspruch 11, **dadurch gekennzeichnet, dass** das Zuordnen des erfassten Absorptions- oder Reflektionsspektrums zu dem ähnlichsten Absorptions- oder Reflektionsspektrum der Ranzigkeitsindextabelle durch Vergleich von zumindest einem aus einem Mittelwert, einer Bandbreite oder einzelner Frequenzbänder der Absorptions- oder Reflektionsspektren erfolgt.

13. Verfahren gemäß einem der Ansprüche 11 oder 12, **gekennzeichnet durch** das Ausscheiden der Ölfrucht, Nuss oder des Samens (2) wenn der ermittelte Ranzigkeitsindexwert einen Schwellenwert überschreitet.

14. Vorrichtung (1) zur Detektion ranziger Ölfrüchte, Nüsse oder Samen (2), mit einer Lichtquelle (3), einem Fotosensor (4), einer Rechnereinheit (5) und einer Sortiereinheit (6), wobei
die Lichtquelle (3) dazu ausgebildet ist die Ölfrucht, Nuss oder den Samen (2) zu bestrahlen, der Fotosensor (4) mit der Rechnereinheit (3) verbunden und dazu ausgebildet ist, ein Absorptions- oder Reflektionsspektrum des von der Ölfrucht, Nuss oder dem Samen (2) reflektierten oder durch die Ölfrucht, Nuss oder den Samen (2) hindurch transmittierten Lichts zu erfassen und an die Rechnereinheit (5) zu übermitteln, und die Sortiereinheit (6) mit der Rechnereinheit (5) verbunden ist, wobei die Rechnereinheit (5) so konfiguriert ist, dass sie die Sortiereinheit (6) durch Abarbeiten des Verfahrens gemäß einem der Ansprüche 11 bis 13 steuert.

## Claims

1. A process for establishing a rancidity index table and allocating a rancidity index value to an absorption or reflection spectrum of oil fruits, nuts and seeds (2), comprising the steps of:
- irradiating a sample of an oil fruit, a nut or a seed (2) with a light source (3),
- projecting the reflected and/or transmitted light onto a photosensor (4),
- detecting the absorption or reflection spectrum in a wavelength range from 900 to 2500 nm, preferably from 900 to 1700 nm, more preferably from 1000 to 1500 nm, by means of the photosensor (4),
- extracting ingredients of the sample by sample preparation techniques based on a determination of volatile compounds from a vapour space above the sample, preferably by solid phase microextraction,
- separating volatile components of the sample by applying gas-chromatographic techniques,
- identifying separated volatile components of the sample by mass spectroscopic detection of constituents of the volatile components which are relevant for lipid oxidation,
**characterized in that** the process comprises the steps of
- determining a rancidity index value of the sample from identified volatile components of the sample,
- allocating at least individual characteristic wavelengths or wavelength ranges of the detected absorption or reflection spectrum of the sample to the rancidity index value,
- repeating the previous steps for a representative number of samples and forming a rancidity table from the determined rancidity index values and allocated absorption or reflection spectra or, respectively, characteristic wavelengths or wavelength ranges of the detected absorption or reflection spectra.

2. A process according to claim 1, **characterized in that** identifying the volatile components of the sample by mass spectroscopic detection of constituents relevant for lipid oxidation comprises identifying one or more groups of substances/functional groups selected from:
- hydroperoxides
- cyclic hydroperoxides
- saturated, mono- and di-unsaturated aldehydes
- hydrocarbons (alkanes, alkenes)
- alcohols (saturated and unsaturated)
- ketones (saturated and unsaturated)
- short chain fatty acids
- alkyl furans.

3. A process according to claim 1 or 2, **characterized in that** the identification of the volatile components of the sample by mass spectroscopic detection of constituents relevant for lipid oxidation occurs by establishing chromatograms at mass/charge ratios selected in a range of between 20 and 300, preferably at at least one mass/charge ratio selected from 43, 44, 55, 56, 57, 60, 70, 71, 73, 74, 81, 83, 97.

4. A process according to claim 3, **characterized by** the creation of a fatty acid chromatogram at a mass/charge ratio characteristic of fatty acids, in particular at a mass/charge ratio of 60.

5. A process according to claim 4, **characterized by** the determination of a fatty acid index value by integration across at least a portion of the fatty acid chromatogram.

6. A process according to any of claims 3 to 5, **characterized by** the creation of an aldehyde chromatogram at a mass/charge ratio characteristic of aldehydes, in particular at a mass/charge ratio of 44.

7. A process according to claim 6, **characterized by** the determination of an aldehyde index value by integration across at least a portion of the aldehyde chromatogram.

8. A process according to claims 5 and 7, **characterized in that** the determination of a rancidity index value occurs by forming the sum of the aldehyde index value and the fatty acid index value.

9. A process according to any of the preceding claims, **characterized in that** the allocation of the detected absorption or reflection spectra of the samples to the rancidity index values is effected by allocating the rancidity index values to at least one of an average, a bandwidth or individual frequency bands of the detected absorption or reflection spectra.

10. A process according to any of the preceding claims, **characterized in that** the detection of the absorption or reflection spectrum is effected by hyperspectral detection by means of the photosensor (4).

11. A process for the detection of rancidity in an oil fruit, a nut or a seed (2), comprising the steps of:
- irradiating the oil fruit, nut or seed (2) with at least one light source (3),
- projecting the reflected and/or transmitted light onto a photosensor (4),
- detecting an absorption or reflection spectrum in a wavelength range from 900 to 2500 nm, preferably from 900 to 1700 nm, more preferably from 1000 to 1500 nm, by means of the photosensor (4),
**characterized in that** the process comprises the steps of
- providing a rancidity index table according to any of claims 1 to 10 which contains the rancidity index values and the allocated absorption or reflection spectra or, respectively, characteristic wavelengths or wavelength ranges of the absorption or reflection spectrum,
- allocating the detected absorption or reflection spectrum or, respectively, characteristic wavelengths or wavelength ranges of the detected absorption or reflection spectrum to an absorption or reflection spectrum of the rancidity index table which is most similar to the detected absorption or reflection spectrum or, respectively, to characteristic wavelengths or wavelength ranges of the absorption or reflection spectrum,
- determining the rancidity index value allocated to the most similar absorption or reflection spectrum or, respectively, characteristic wavelengths or wavelength ranges of the absorption or reflection spectrum.

12. A process according to claim 11, **characterized in that** the allocation of the detected absorption or reflection spectrum to the most similar absorption or reflection spectrum of the rancidity index table is effected by comparing at least one of an average, a bandwidth or individual frequency bands of the absorption or reflection spectra.

13. A process according to any of claims 11 or 12, **characterized by** the segregation of the oil fruit, nut or seed (2), if the determined rancidity index value exceeds a threshold value.

14. A device (1) for the detection of rancid oil fruits, nuts or seeds (2), comprising a light source (3), a photosensor (4), a computer unit (5) and a sorting unit (6), wherein
the light source (3) is designed for irradiating the oil fruit, nut or seed (2), the photosensor (4) is connected to the computer unit (3) and designed for detecting an absorption or reflection spectrum of the light reflected from the oil fruit, nut or seed (2) or transmitted through the oil fruit, nut or seed (2) and transmitting it to the computer unit (5), and the sorting unit (6) is connected to the computer unit (5), the computer unit (5) being configured such that it controls the sorting unit (6) by executing the process according to any of claims 11 to 13.

## Revendications

1. Procédé pour établir une table d'indice de rancissement et associer une valeur d'indice de rancissement à un spectre d'absorption ou de réflexion de fruits oléagineux, de noix et de graines (2), comprenant les étapes consistant à :
• irradier un échantillon d'un fruit oléagineux, d'une noix ou d'une graine (2) avec une source de lumière (3) ;
• projeter la lumière réfléchie et/ou transmise sur un photocapteur (4) ;
• détecter le spectre d'absorption ou de réflexion dans une plage de longueurs d'onde qui est comprise entre 900 nm et 2500 nm, de préférence entre 900 nm et 1700 nm, et de manière encore plus préférée entre 1000 nm et 1500 nm, à l'aide du photocapteur (4) ;
• extraire des composants de l'échantillon en utilisant des techniques de préparation d'échantillon basées sur une détermination des composés volatils à partir d'un espace de vapeur qui est situé au-dessus de l'échantillon, et de préférence en réalisant une micro-extraction en phase solide ;
• séparer les composants volatils de l'échantillon en utilisant des techniques de chromatographie en phase gazeuse ; et
• identifier les composants volatils séparés de l'échantillon en effectuant une détection par spectroscopie de masse des constituants des composants volatils pertinents pour l'oxydation des lipides,
**caractérisé en ce que** le procédé comprend les étapes consistant à :
• déterminer une valeur d'indice de rancissement de l'échantillon à partir des composants volatils identifiés de l'échantillon ;
• associer à la valeur d'indice de rancissement au moins des longueurs d'onde ou des plages de longueurs d'onde caractéristiques individuelles du spectre d'absorption ou de réflexion détecté de l'échantillon ; et
• répéter les étapes précédentes pour un nombre représentatif d'échantillons et établir une table de rancissement à partir des valeurs d'indice de rancissement déterminées ainsi que des spectres d'absorption ou de réflexion associés ou des longueurs d'onde ou des plages de longueurs d'onde caractéristiques des spectres d'absorption ou de réflexion détectés.

2. Procédé selon la revendication 1, **caractérisé en ce que** l'identification des composants volatils de l'échantillon réalisée en effectuant une détection par spectroscopie de masse des constituants pertinents pour l'oxydation des lipides comprend l'identification d'un ou de plusieurs groupe(s) de substances/fonctions choisi(s) parmi :
• les hydroperoxydes ;
• les hydroperoxydes cycliques ;
• les aldéhydes saturés, mono-insaturés et di-insaturés ;
• les hydrocarbures (alcanes, alcènes) ;
• les alcools (saturés et insaturés) ;
• les cétones (saturées et non saturées) ;
• les acides gras à chaîne courte ; et
• les alkyles furanes.

3. Procédé selon la revendication 1 ou 2, **caractérisé en ce que** l'identification des composants volatils de l'échantillon réalisée en effectuant une détection par spectroscopie de masse de constituants pertinents pour l'oxydation des lipides est effectuée en établissant des chromatogrammes à des rapports de masse/charge qui sont choisis dans une plage comprise entre 20 et 300, de préférence à au moins un rapport de masse/charge qui est choisi parmi 43, 44, 55, 56, 57, 60, 70, 71, 73, 74, 81, 83, 97.

4. Procédé selon la revendication 3, **caractérisé par** l'établissement d'un chromatogramme d'acides gras à un rapport de masse/charge caractéristique des acides gras, notamment à un rapport de masse/charge égal à 60.

5. Procédé selon la revendication 4, **caractérisé par** la détermination d'une valeur d'indice d'acide gras qui est réalisée en effectuant une intégration du chromatogramme d'acide gras sur au moins une zone.

6. Procédé selon l'une quelconque des revendications 3 à 5, **caractérisé par** l'établissement d'un chromatogramme d'aldéhydes à un rapport de masse/charge caractéristique des aldéhydes, notamment à un rapport de masse/charge égal à 44.

7. Procédé selon la revendication 6, **caractérisé par** la détermination d'une valeur d'indice d'aldéhyde qui est réalisée en effectuant une intégration du chromatogramme d'aldéhyde sur au moins une zone.

8. Procédé selon les revendications 5 et 7, **caractérisé en ce que** la détermination d'une valeur d'indice de rancissement est calculée en effectuant la somme de la valeur d'indice d'aldéhyde et de la valeur d'indice d'acide gras.

9. Procédé selon l'une quelconque des revendications précédentes, **caractérisé en ce que** l'association des spectres d'absorption ou de réflexion détectés des échantillons avec les valeurs d'indice de rancissement est réalisée en associant les valeurs d'indice de rancissement à au moins une valeur parmi une valeur moyenne, une largeur de bande ou des bandes de fréquences individuelles des spectres d'absorption ou de réflexion détectés.

10. Procédé selon l'une quelconque des revendications précédentes, **caractérisé en ce que** la détection du spectre d'absorption ou de réflexion à l'aide du photocapteur (4) est réalisée en effectuant une détection hyperspectrale.

11. Procédé de détection du rancissement d'un fruit oléagineux, d'une noix ou d'une graine (2), comprenant les étapes suivantes :
• exposer le fruit oléagineux, la noix ou la graine (2) à au moins une source de lumière (3) ;
• projeter la lumière réfléchie et/ou transmise sur un photocapteur (4) ; et
• détecter, à l'aide du photocapteur (4), un spectre d'absorption ou de réflexion dans une plage de longueurs d'onde qui est comprise entre 900 nm et 2500 nm, de préférence entre 900 nm et 1700 nm, et de manière encore plus préférée entre 1000 nm et 1500 nm,
**caractérisé en ce que** le procédé comprend les étapes consistant à :
• établir une table d'indice de rancissement selon l'une quelconque des revendications 1 à 10 qui contient les valeurs d'indice de rancissement ainsi que les spectres d'absorption ou de réflexion associés ou les longueurs d'onde ou les plages de longueurs d'onde caractéristiques du spectre d'absorption ou de réflexion ;
• associer le spectre d'absorption ou de réflexion détecté ou des longueurs d'onde ou des plages de longueurs d'onde caractéristiques du spectre d'absorption ou de réflexion détecté à un spectre d'absorption ou de réflexion de la table d'indice de rancissement le plus similaire du spectre d'absorption ou de réflexion détecté ou à des longueurs d'onde ou des plages de longueurs d'onde caractéristiques du spectre d'absorption ou de réflexion ; et
• déterminer la valeur de l'indice de rancissement associée au spectre d'absorption ou de réflexion le plus similaire ou aux longueurs d'onde ou aux plages de longueurs d'onde caractéristiques du spectre d'absorption ou de réflexion.

12. Procédé selon la revendication 11, **caractérisé en ce que** l'association du spectre d'absorption ou de réflexion détecté au spectre d'absorption ou de réflexion le plus similaire de la table d'indice de rancissement est réalisée en effectuant une comparaison d'au moins une valeur parmi une valeur moyenne, une largeur de bande ou des bandes de fréquence individuelles des spectres d'absorption ou de réflexion.

13. Procédé selon l'une quelconque des revendications 11 ou 12, **caractérisé par** l'élimination du fruit oléagineux, de la noix ou de la graine (2) lorsque la valeur de l'indice de rancissement déterminée est supérieure à une valeur de seuil.

14. Dispositif (1) de détection de fruits oléagineux, de noix ou de graines (2) rances, comprenant une source de lumière (3), un photocapteur (4), une unité de calcul (5) ainsi qu'une unité de tri (6), la source de lumière (3) étant conçue de manière à irradier le fruit oléagineux, la noix ou la graine (2), le photocapteur (4) étant relié à l'unité de calcul (3) et étant conçu de manière à générer un spectre d'absorption ou de réflexion de la lumière émise par le fruit oléagineux, la noix ou la graine (2), ou transmise à travers le fruit oléagineux, la noix ou la graine (2), et à transmettre celui-ci à l'unité de calcul (5), et l'unité de tri (6) étant reliée à l'unité de calcul (5), l'unité de calcul (5) étant configurée de manière à commander l'unité de tri (6) en exécutant le procédé selon l'une quelconque des revendications 11 à 13.
